# FASCICULE DE BREVET EUROPEEN

(11) **EP 0 699 349 B1**
(45) Date de publication et mention de la délivrance du brevet: **10.10.2007**
(21) Numéro de dépôt: 95914390.0
(22) Date de dépôt: 21.03.1995
(51) Int. Cl.: H01M 10/40, H01M 6/18, C01B 21/093, C07C 317/04, C23F 11/16, C07C 311/48

(54) **MATERIAU A CONDUCTION IONIQUE PRESENTANT DE BONNES PROPRIETES ANTI-CORROSION**
IONENLEITENDES MATERIAL MIT GUTEN KORROSIONSHEMMENDEN EIGENSCHAFTEN
IONIC CONDUCTING MATERIAL HAVING GOOD ANTICORROSIVE PROPERTIES

(30) Priorité: 21.03.1994 FR 9403276; 21.03.1994 FR 9403277
(43) Date de publication de la demande: 06.03.1996
(73) Titulaire: CENTRE NATIONAL DE LA RECHERCHE SCIENTIFIQUE (CNRS), 75016 Paris (FR); HYDRO-QUEBEC, Montréal Québec H2Z 1A4 (CA)
(72) Inventeur: MICHOT, Christophe, F-38000 Grenoble (FR); ARMAND, Michel, F-38410 Saint-Martin-d'Uriage (FR); SANCHEZ, Jean-Yves, F-38330 Saint-Ismier (FR); CHOQUETTE, Yves, Sainte-Julie, Québec J3E 1P4 (CA); GAUTHIER, Michel, Varennes, Québec J3X 1S1 (CA)
(74) Mandataire: Sueur, Yvette
(86) Numéro de dépôt international: PCT/FR1995/000343
(87) Numéro de publication internationale: WO 1995/026056

(56) Documents cités:
- EP-A- 0 096 629
- EP-A- 0 466 483
- EP-A- 0 626 734
- WO-A-92/02966
- WO-A-93/09092
- PATENT ABSTRACTS OF JAPAN vol. 18, no. 63 (E-1500) 2 Février 1994 & JP,A,05 283 086 (HITACHI MAXELL LTD) 29 Octobre 1993 cité dans la demande
- CHEMICAL ABSTRACTS, vol. 115, no. 19, 11 Novembre 1991, Columbus, Ohio, US; abstract no. 207464, DESMARTEAU 'novel layered structures in metal salts of bis(sulfonyl)methanes' & EUR. J. SOLID STATE INORG. CHEM., vol.28, no.5, 1991 pages 905 - 917
- CHEMICAL ABSTRACTS, vol. 118, no. 23, 7 Juin 1993, Columbus, Ohio, US; abstract no. 234164, VIJ,ASHWANI 'DIMETHYLTIN(IV)BIS (FLUOROSULFONYL)IMIDE' & MAIN GROUP MET. CHEM., vol.15, no.2, 1992 pages 81 - 87
- CHEMICAL ABSTRACTS, vol. 116, no. 12, 30 Mars 1992, Columbus, Ohio, US; abstract no. 128072, YAGUPOL' SKII 'TRIS( ***FLUOROSULFONYL*** )METHANIDE -C(SO2F)3 - AN EFFECTIVE STABILIZING NONNUCLEOPHILIC ***CARBANION***' & ZH. OBSHCH. KHIM., vol.61, no.7, 1 Juillet 1991 pages 1512 - 1518
- PATENT ABSTRACTS OF JAPAN vol. 18, no. 504 (E-1608) 21 Septembre 1994 & JP,A,06 176 769 (HITACHI MAXELL LTD) 24 Juin 1994
- CHEMICAL ABSTRACTS, vol. 122, no. 19, 8 Mai 1995, Columbus, Ohio, US; abstract no. 239291, MALETINA 'PHENYLBIS(FLUOROSULFONYL)- AND P HENYLBIS(TRIFLUOROMETHYLSULFONYL)METHYLIOD ONIUM TRIFLATES' & J.FLUORINE CHEM.(1995),70(1),85-8, 30 Janvier 1995
- CHEMICAL ABSTRACTS, vol. 86, no. 13, 28 Mars 1977, Columbus, Ohio, US; abstract no. 89981, FROBOESE,R. 'TRANSITION METAL BIS(FLUOROSULFONYL)AMIDES' & Z.NATURFORSCH.,B:ANORG.CHEM.,ORG.CHEM., vol.31B, no.11, 1976 pages 1497 - 1500
- PATENT ABSTRACTS OF JAPAN vol. 18, no. 139 (E-1519) 8 Mars 1994 & JP,A,05 326 016 (SANYO ELECTRIC CO LTD) 10 Décembre 1993

## Description

La présente invention concerne un composé pour un matériau à conduction ionique, sa préparation et les utilisations du matériau.

Les systèmes électrochimiques de stockage d'énergie, par exemple les batteries ou les supercapacités, qui fonctionnent avec des tensions élémentaires élevées exigent des électrolytes qui ont un domaine de stabilité étendu. De tels électrolytes sont obtenus par dissolution d'un ou plusieurs solutés (1/mM')⁺X'⁻ dans un solvant liquide dipolaire, un polymère solvatant ou leurs mélanges. M' est un cation de valence m tel qu'un proton, un cation dérivé d'un métal (par exemple Li, Na, K, Mg, Ca, Cu, Zn, La) ou un cation organique tel qu'un ion ammonium, un ion guanidinium, un ion phosphonium ou un ion sulfonium. Les composés ioniques (1/mM')⁺X'⁻ dans lesquels l'anion X'⁻ possède une charge délocalisée, présentent une conductivité ionique élevée. Parmi les anions X'- à charge délocalisée, on peut citer I⁻, ClO₄⁻, AsF₆⁻, PF₆⁻, R_{F}SO₃⁻, (R_{F}SO₂)₂N⁻, R¹CO(CF₃SO₂)₂C⁻, R¹SO₂(CF₃SO₂)₂C-, R_{F} représentant un radical perfluoroalkyle ou un radical perfluoroaryle, R¹ représentant un atome d'hydrogène, un radical alkyle, un radical oxa-alkyle, un radical aza-alkyle, un radical aryle, un radical perfluoroalkyle ou un radical perfluoroaryle.

Bien que les composés précités aient des conductivités ioniques élevées, leur usage présente des inconvénients. Les ions iodures sont facilement oxydables. Les dérivés de l'arsenic sont toxiques et les perchlorates sont explosifs. Les anions tels que PF₆-, susceptibles de libérer facilement un acide de Lewis, (PF₆⁻ →PF₅) compromettent la stabilité des électrolytes correspondants par des réactions provoquant la formation d'espèces électrophiles de type carbocation.

Les anions contenant des groupements perfluorosulfonyles R_{F}SO₂-, en particulier les perfluorosulfonates R_{F}SO₃- et les perfluorosulfonylimidures (R_{F}SO₂)₂N- sont stables et présentent une faible toxicité et l'utilisation des composés ioniques correspondants s'est généralisée, notamment pour les générateurs électrochimiques comprenant des électrodes négatives constituées par du lithium métallique, un alliage de lithium ou un composé d'insertion lithium-carbone. La préparation de ces composés ioniques est toutefois très onéreuse, et tout particulièrement la fabrication des composés contenant au moins deux groupements perfluorosulfonyles. En outre, ces composés ont une masse moléculaire élevée et la fraction massique pour une molalité donnée dans un solvant est importante.

On connaît les composés répondant à la formule (1/mM)⁺[FSO₂NSO₂F]⁻ pour (1/mM)⁺ = H⁺, K⁺, Cs⁺, Rb+, Ag+, (CH₃)₄N⁺ [J.K. Ruff, Inorg. Chem. 4, 1446, (1965)]. De manière générale, un sel constitue un électrolyte d'autant meilleur que la basicité de son anion est plus faible. La basicité de l'anion [FSO₂NSO₂F]⁻ est a priori plus élevée que celle d'un anion [R_{F}SO₂NSO₂R_{F}]⁻ dans lequel R_{F} est un groupement perfluoroalkyle, du fait de la rétrocession des doublets libre du fluor sur l'atome de soufre. De nombreuses publications font état du fait que le remplacement d'un groupement perfluoroalkyle pour un atome de fluor dans un composé organique présentant un caractère acide diminue la force de l'acide. (G. Paprott & K. Seppelt, J. Am. Chem. Soc., 106, 4060 (1984) ; E. D. Laganis, D. M. Lema, J. Am. Chem. Soc., 102, 6634 (1984) ; F. J. Bordwell, J.C. Branca, et al., J. Org. Chem., 53, 780, (1988).

En outre, il est connu que l'atome de fluor d'une liaison F-S est particulièrement labile, et notamment hydrolysable en présence d'eau ou de bases nucléophiles. Du fait de ces inconvénients, l'utilisation de l'acide [FSO₂NSO₂F]H comme électrolyte protonique dans les piles à combustible a été déconseillée ([M. Razak, et al., J. Appl. Electrochem., 17(5), 1057 (1987)]. Au contraire, la stabilité des composés [R_{F}SO₂NSO₂R_{F}]H tels que H(CF₃SO₂)₂N ou H(CF₃SO₂NSO₂C₄F₉) a été démontrée [M. Razak, et al., précité ; M. Razak, et al., J. Appl. Electrochem., 136, 385 (1989)].

Il est également connu que les composés H(FSO₂)₃C s'hydrolysent spontanément, alors que leurs homologues (l/mM')⁺ [(R_{F}SO₂)₃C]⁻ ont été proposés comme solutés d'électrolytes pour des générateurs électrochimiques. Toutefois, de même que pour les imidures, la masse moléculaire et les coûts de fabrication des composés (1/mM)⁺[(R_{F}SO₂)₃C]⁻ sont élevés et rendent leur utilisation peu intéressante.

JP-A-05 283 086 concerne une batterie dans laquelle l'électrolyte contient un éther cyclique comme solvant et un sel comprenant au moins deux groupements R_{F}SO₂, R_{F} étant un atome de fluor ou un groupe fluoroalkyle. L'utilisation de sels contenant deux groupements fluoroalkyles est décrite et illustrée par des exemples concrets relatifs au bis(trifluorométhanesulfonyl) méthylure de lithium. Il est expliqué qu'un sel du type (CF₃SO₂)₂NLi ou (CF₃SO₂)₃CLi donne une conductivité supérieure par rapport à un sel CF₃SO₃Li par le fait que la présence d'un seul groupe électroattracteur sur l'atome voisin de l'atome de lithium dans CF₃SO₃Li augmente la densité électronique sur cette atome, en l'occurence l'oxygène, et rend donc plus difficile l'ionisation, c'est-à-dire la libération de Li⁺, alors que dans un composé (CF₃SO₂)₂NLi ou (CF₃SO₂)₃CLi, la présence des deux groupements électro-attracteurs sur l'atome voisin du lithium diminue la densité électronique sur cet atome et favorise donc la libération de l'ion Li⁺. Aucune information n'est donnée sur des sels comprenant un ou deux groupements FSO₂. En outre, les conclusions tirées de la comparaison entre un composé R_{F}SO₃Li et un composé (R_{F}SO₂)₂NLi ou (R_{F}SO₂)₃CLi lorsque R_{F} est CF₃ ne peuvent être simplement extrapolées aux composés correspondants dans lesquels R_{F} est F. En effet, un composé FSO₃Li n'est pas stable en solution dans un éther cyclique, dans lequel il se décompose pour donner LiF et SO₃, provoquant ainsi une polymérisation de l'éther, en particulier dans le cas des acétals cycliques. Ce composé n'était donc absolument pas utilisable comme sel dans un éther. Par voie de conséquence, il n'était pas évident que l'amélioration de la conductivité par le remplacement de CF₃SO₃Li (qui est un sel utilisable, même s'il ne s'agit pas du plus performant) par (CF₃SO₂)₂NLi ou (CF₃SO₂)₃CLi pouvait être transposée au cas du remplacement de FSO₃Li (qui est un sel inutilisable) par (FSO₂)₂NLi ou (FSO₂)₃CLi.

Les inventeurs ont trouvé que des matériaux à conduction ionique présentant d'excellentes propriétés de conductivité et de stabilité peuvent être obtenus à partir de composés ioniques fluorés qui comprennent au moins un groupement dans lequel un atome de fluor est directement lié à un hétéroatome.

L'invention concerne le composé défini à la revendication 4, le procédé de préparation du composé défini à la revendication 1 et le matériau à conduction ionique défini à la revendication 7.

Alors que les publications de l'art antérieur suggéraient que le remplacement d'un groupe perfluoroalkyle par un atome de fluor dans un sel provoquait une diminution de la dissociation du dit sel, et qu'en outre, une liaison F-S était moins stable qu'une liaison C-F, toutes choses étant identiques par ailleurs, les inventeurs ont trouvé de manière surprenante que les composés ioniques utilisés dans les matériaux à conduction ionique de la présente invention présentent une grande stabilité chimique aussi bien qu'électrochimique dans les milieux aprotiques, malgré l'existence de liaisons S-F. De tels composés présentent par conséquent un large domaine de stabilité vis à vis des phénomènes d'oxydoréduction. En outre, la conductivité de ces composés ioniques en solution dans des solvants aprotiques ou dans des polymères solvatants ou dans leurs mélanges est au moins comparable, voire supérieure à celles des composés ioniques utilisés classiquement ou à celles des dérivés de [R_{F}SO₂NSO₂R_{F}]⁻. De plus, les composés ioniques de l'invention présentent une masse moléculaire plus faible que celle des composés perfluoroalkylés correspondants et leur préparation est plus économique du fait qu'elle part de produits industriels.

Lorsque le substituant Z est différent d'un atome de fluor, il peut être choisi pour conférer au composé ionique de l'invention la ou les propriétés additionnelles en vue de son utilisation. Le choix pour Z est par conséquent très large.

De manière générale, Z peut être choisi parmi les radicaux alkyles en C₁-C₃₀ ou perhaloalkyles en C₁-C₈, les radicaux aryles ou perhaloaryles en C₆-C₁₂, les radicaux arylalkyles, les radicaux oxaalkyles, azaalkyles, thiaalkyles et les hétérocycles. Plus particulièrement, lorsque Z est un radical alkyle, un radical aryle-alkyle, ou un radical perhaloalkyle ayant plus de 4 atomes de carbone, le composé ionique de la présente invention présente des propriétés tensioactives.

Lorsque Z représente un groupe mésomorphe, le composé ionique de l'invention présente les propriétés d'un cristal liquide.

La fonction polymérisable du substituant Z peut être une fonction polymérisable par exemple par voie radicalaire, par voie anionique ou par une réaction de type Vandenberg.

Lorsque Z comporte des insaturations éthyléniques, par exemple -C=C-, -C=C-C=O, -C=SO₂-, -C=CΦ, le composé de l'invention peut être polymérisé.

Lorsque Z comporte au moins un groupe fonctionnel condensable tel que par exemple un groupe -NH₂ porté par un groupement aromatique, ou un groupe -OH ou -COOH ou -N=C=O, le composé ionique de l'invention peut être incorporé dans un réseau obtenu par polycondensation.

Lorsque Z comporte un groupe dissociable tel que par exemple un groupe peroxyde -O-O-, un groupe diazoïque -N=N, un groupe azo N₂=CH-, un groupe -SO₂N₃, un groupe disulfure - S-S-, le composé ionique de l'invention peut être utilisé comme initiateur radicalaire.

Le groupe Z peut être constitué par une chaîne polymérique. Le composé ionique de l'invention peut alors constituer un polyélectrolyte.

Le groupe Z peut être un phénol encombré ou une quinone. Le composé de l'invention constitue alors un piège à radicaux libres et présente des propriétés anti-oxydantes.

Lorsque Z est un groupe chromophore, par exemple la Rhodamine B, le composé ionique de l'invention est un colorant.

Lorsque Z comporte une fonction amide, nitrile ou ester cyclique, le composé ionique de l'invention constitue un dipôle dissociant.

Z peut également comporter un couple redox tel que par exemple un disulfure, un thioamide, un ferrocène, une phénothiazine, un bis(dialkylamino)aryle, un nitroxyde, un imide aromatique.

Z peut également être un polymère conducteur électroniquement dopé ou autodopé.

Z peut également constituer un ligand complexant ou un zwitterion.

Z peut en outre être un alcoxysilane hydrolysable, un acide aminé, un polypeptique optiquement ou biologiquement actif.

Z peut également être choisi parmi les groupes R³-CFX-, R³-O-CF₂-CFX-, R¹R²N-CO-CFX- ou R¹R²N-SO₂-(CF₂)ₙ-CFX- avec n = 1, 2 ou 3, dans lesquels :
- X représente F, Cl, H ou R_{F} ;
- les radicaux R¹, R² et R³, identiques ou différents, sont choisis parmi les radicaux organiques non perfluorés polymérisables ;
- R_{F} est choisi parmi les radicaux perfluoroalkyles et les radicaux perfluoroaryles.

Parmi les groupements R_{F} du type perfluoroalkyle, on préfère les radicaux perfluoroalkyle ayant de 1 à 8 atomes de carbone, et plus particulièrement les radicaux perfluoroalkyles ayant de 1 à 4 atomes de carbone. On peut citer les radicaux CF₃-, C₂F₅-, n-C₃F₇-, n-C₄F₉-.

Parmi les groupements R_{F} du type perfluoroaryle, on préfère les radicaux perfluoroaryle ayant de 6 à 8 atomes de carbone, par exemple le radical perfluorophényle.

Les groupements organiques non perfluorés polymérisables R¹, R² et R³ permettent des réactions de polymérisation par voie radicalaire, anionique, cationique ou stéréospécifique, ou par polycondensation. Ils peuvent être choisis parmi ceux qui comportent des doubles liaisons, par exemple des doubles liaisons du type vinyle, allyle, vinylbenzyle ou acryloyle. Ils peuvent également être choisis parmi ceux qui comportent des fonctions oxirane ou oxétane. En outre, ils peuvent être choisis parmi ceux qui comportent des fonctions alcool, thiol, arylamine, isocyanate ou trialcoxysilane. Ils peuvent également être choisis parmi ceux qui comportent des fonctions permettant une électropolymérisation.

Un matériau à conduction ionique de la présente invention comprend au moins un composé ionique tel que décrit ci-dessus et au moins un solvant aprotique.

Le solvant peut être un solvant liquide aprotique, choisi par exemple parmi les éthers linéaires et les éthers cycliques, les esters, les nitriles, les dérivés nitrés, les amides, les sulfones, les sulfolanes et les sulfamides. Les solvants particulièrement préférés sont le diéthyléther, le diméthoxyéthane, le tétrahydrofurane, le dioxane, le diméthyltétrahydrofurane, le formiate de méthyle ou d'éthyle, le carbonate de propylène ou d'éthylène, les butyrolactones, l'acétonitrile, le benzonitrile, le nitrométhane, le nitrobenzène, la diméthylformamide, la diéthylformamide, la N-méthylpyrrolidone, la diméthylsulfone, la tétraméthylène sulfone et la tétraéthylsulfonamide.

Le solvant peut également être choisi parmi les polymères solvatants, réticulés ou non, portant ou non des groupes ioniques greffés. Un polymère solvatant est un polymère qui comporte des unités solvatantes contenant au moins un hétéroatome choisi parmi le soufre, l'oxygène, l'azote et le fluor. A titre d'exemple de polymères solvatants, on peut citer les polyéthers de structure linéaire, peigne ou à blocs, formant ou non un réseau, à base de poly(oxyde d'éthylène), ou les copolymères contenant le motif oxyde d'éthylène ou oxyde de propylène ou allylglycidyléther, les polyphosphazènes, les réseaux réticulés à base de polyéthylène glycol réticulé par des isocyanates ou les réseaux obtenus par polycondensation et portant des groupements qui permettent l'incorporation de groupements réticulables. On peut également citer les copolymères à blocs dans lesquels certains blocs portent des fonctions qui ont des propriétés rédox. Bien entendu, la liste ci-dessus n'est pas limitative, et tous les polymères présentant des propriétés solvatantes peuvent être utilisés.

Un matériau à conduction ionique de la présente invention peut comprendre simultanément un solvant liquide aprotique choisi parmi les solvants liquides aprotiques cités ci-dessus et un solvant polymère solvatant. Il peut comprendre de 2 à 98% de solvant liquide.

Le solvant d'un matériau à conduction ionique de la présente invention peut également être consitué essentiellement par un polymère polaire non solvatant comprenant des unités contenant au moins un hétéroatome choisi parmi le soufre, l'oxygène, l'azote et le fluor, et par un liquide aprotique choisi parmi les solvants liquides aprotiques mentionnés ci-dessus. A titre d'exemple de polymère polaire non solvatant, on peut citer un poly(acrylonitrile), un poly (fluorovinylidène) ou une poly (N-vinylpyrrolidone). La proportion de liquide aprotique dans le solvant peut varier de 2% (correspondant à un solvant plastifié) à 98% (correspondant à une solvant gélifié).

Un matériau à conduction ionique de la présente invention peut contenir en outre un sel utilisé classiquement dans l'art antérieur pour l'élaboration d'un matériau à conduction ionique. Dans un tel cas, le composé ionique de l'invention joue égalemnt le rôle d'additif de passivation du collecteur de la cathode, par exemple lorsque le matériau à conduction ionique est utilisé dans un générateur au lithium rechargeable, dont la cathode a un collecteur en aluminium. Parmi les sels utilisables en mélange avec un composé ionique selon l'invention, on préfère tout particulièrement un sel choisi parmi les perfluoroalcanesulfonates, les bis(perfluoroalkylsulfonyl) imidures, les bis(perfluoroalkylsulfonyl) méthanes et les tris(perfluoroalkylsulfonyl)méthanes.

Bien entendu, un matériau à conduction ionique de l'invention peut contenir en outre les additifs utilisés de manière classique dans ce type de matériau, et notamment un plastifiant, une charge, d'autres sels... etc.

Un composé ionique Li⁺[FSO₂NSO₂Z]⁻ selon la présente invention, dans lequel Z représente un atome de fluor ou un radical perfluoroalkyle R_{F}, peut être préparé en faisant réagir l'acide correspondant [FSO₂NSO₂Z]H dans un solvant aprotique non réactif sur un sel du cation Li qui est choisi de manière à former au cours de la réaction, un acide volatil ou insoluble dans le milieu réactionnel et dont la basicité est suffisamment faible pour ne pas affecter la liaison S-F. La réaction peut avoir lieu avantageusement suivant le schéma réactionnel suivant :

[FSO₂NSO₂Z]H + LiF → Li+[FSO₂NSO₂Z]⁻ + HF ↗

Le solvant aprotique peut être choisi parmi les nitriles, les nitroalcanes, les esters et les éthers. L'acétonitrile est un solvant particulièrement préféré en raison de sa volatilité et de sa stabilité.

Le sel utilisé pour réagir avec l'acide de départ et susceptible de libérer un acide volatil peut être choisi parmi les fluorures, les chlorures, les acétates, les trifluoroacétates. Les fluorures, qui ne réagissent pas sur le groupement fluorosulfonyle et qui produisent l'acide volatil HF sont particulièrement préférés.

Le sel utilisé pour réagir avec l'acide de départ et qui permet d'obtenir un acide insoluble peut être choisi parmi les sels de diacides organiques ou de polyacides en choisissant une stoechiométrie telle que le produit formé soit un sel acide insoluble dans les solvants aprotiques. Comme exemple de tels sels, on peut citer les oxalates, les malonates, les polyacrylates, les polyméthacrylates réticulés ou non, les polyphosphates et les zéolithes.

Les composés Li⁺[(ZY)₂N]⁻, (dans lesquels Y représente SO₂ et chaque substituant Z représente de manière indépendante un atome de fluor ou un groupement organique perfluoré ou non qui possède éventuellement au moins une fonction polymérisable, l'un au moins des substituants Z représentant un atome de fluor tel que défini précédemment, peuvent être préparés par un procédé analogue à partir de l'acide correspondant H[(ZY)₂N].

Le procédé décrit ci-dessus pour la préparation des composés ioniques de la présente invention est particulièrement intéressant dans la mesure où il permet d'obtenir les sels de lithium qui n'ont pu être obtenus par les procédés de l'art antérieur. Les sels de lithium représentés par la formule Li⁺[(ZY)₂N]⁻ (dans laquelle Y représente SO₂ et chaque substituant Z représente de manière indépendante un atome de fluor ou un groupement organique perfluoré ou non qui possède éventuellement au moins une fonction polymérisable par voie radicalaire, par voie anionique ou par une réaction de type Vandenberg, l'un au moins des substituants Z représentant un atome de fluor), qui ne pouvaient être obtenus par les procédés de l'art antérieur, constituent par conséquent un autre objet de la présente invention.

Un matériau à conduction ionique de la présente invention contenant au moins l'un des composés ioniques précités Li⁺[(ZY)₂N]⁻, (dans lesquels Y représente SO₂ et chaque substituant Z représente de manière indépendante un atome de fluor ou un groupement organique perfluoré ou non qui possède éventuellement au moins une fonction polymérisable, l'un au moins des substituants Z représentant un atome de fluor tel que défini précédemment) en solution dans un solvant aprotique est utilisable comme électrolyte dans un générateur électrochimique au lithium. Par générateur au lithium, on entend un générateur dans lequel l'électrode négative contient du lithium, l'électrode négative pouvant être constituée par du lithium métallique, un alliage de lithium ou encore un lithium ionique inséré, par exemple LiC₆. Dans les deux derniers cas, le générateur est du type rocking chair, dans lequel chacune des électrodes est un composé d'insertion du lithium.

Le matériau peut également être utilisé comme électrolyte dans une supercapacité. Une supercapacité est un système électrochimique comprenant un électrolyte et des électrodes constituées par du carbone ou par un autre matériau inerte de haute surface spécifique, ou encore par un polymère rédox conjugué du type polyacétylène, polyaniline ou polythiophène.

Un matériau de l'invention peut également être utilisé pour le dopage p ou n d'un polymère à conduction électronique. Par exemple, on dope électrochimiquement un film de poly(3-phénylthiophène) dans une solution de l'un des composés ioniques Li⁺[(ZY)₂N]⁻ (dans lesquels Y représente SO₂ et chaque substituant Z représentant de manière indépendante un atome de fluor ou un groupement organique perfluoré ou non qui possède éventuellement au moins une fonction polymérisable, l'un au moins des substituants Z représentant un atome de fluor tel que défini précédemment) dans un solvant liquide et dans un polymère solvatant. Le polymère ainsi dopé peut être utilisé comme matériau d'électrode dans une supercapacité telle que mentionnée ci-dessus.

Un matériau à conduction ionique de l'invention peut également être utilisé dans un système électrochrome.

Un composé ionique de l'invention peut également être utilisé comme constituant d'électrolytes fondus à basse température, et notamment d'électrolytes qui comprennent un polymère permettant de leur conférer des propriétés plastiques ou d'élastomère. Les composés ioniques de l'invention dans lesquels le cation est un imidazolium quaternaire conviennent tout particulièrement pour cette application.

La présente invention est décrite plus en détail à l'aide des exemples ci-dessous, auxquels l'invention n'est cependant pas limitée.

### EXEMPLE 1

### Préparation de bis(fluorosulfonyl)imidure de lithium

On a préparé du bis(fluorosulfonimide) (FSO₂)₂NH par distillation de l'acide fluorosulfonique en présence d'urée, réagissant selon la réaction

3HFSO₃ + CO(NH₂)₂ → (FSO₂)₂NH + CO₂ + HF + (NH₄)HSO₄

Toutes les opérations ont été effectuées dans un appareillage en polymère résistant à l'action de l'acide fluorhydrique. On pourrait également utiliser un appareillage en métal résistant à l'action de l'acide fluorhydrique.

On a dissous 18 g de (FSO₂)₂NH dans 80 ml d'acétonitrile anhydre et on y a ajouté 5 g de fluorure de lithium. Le mélange a été agité pendant 5 heures, puis la suspension obtenue a été centrifugée. Le sel de lithium s'est formé selon la réaction :

[FSO₂NSO₂F]H ⁺ LiF → Li [FSO₂NSO₂F] + HF↗

La solution a été concentrée à l'aide d'un évaporateur rotatif et le sel a été obtenu sous forme anhydre par traitement sous vide primaire.

Le bis(fluorosulfonyl)imidure de lithium (LiFSI) est un solide blanc très soluble dans les solvants aprotiques.

### EXEMPLE 2

On a dissous le sel LIFSI obtenu dans l'exemple 1 dans un polyéther et on a soumis l'électrolyte ainsi obtenu à diverses mesures. Le polyéther utilisé est un copolymère d'oxyde d'éthylène contenant environ 80% en motifs d'oxyde d'éthylène. Un tel copolymère est décrit par exemple dans EP-A-0 013 199 ou dans US-A-4 578 326. La concentration en sel dans l'électrolyte était de 1 mole de LiFSI pour 30 moles d'oxygène des unités solvatantes éther (O/Li = 30/1).

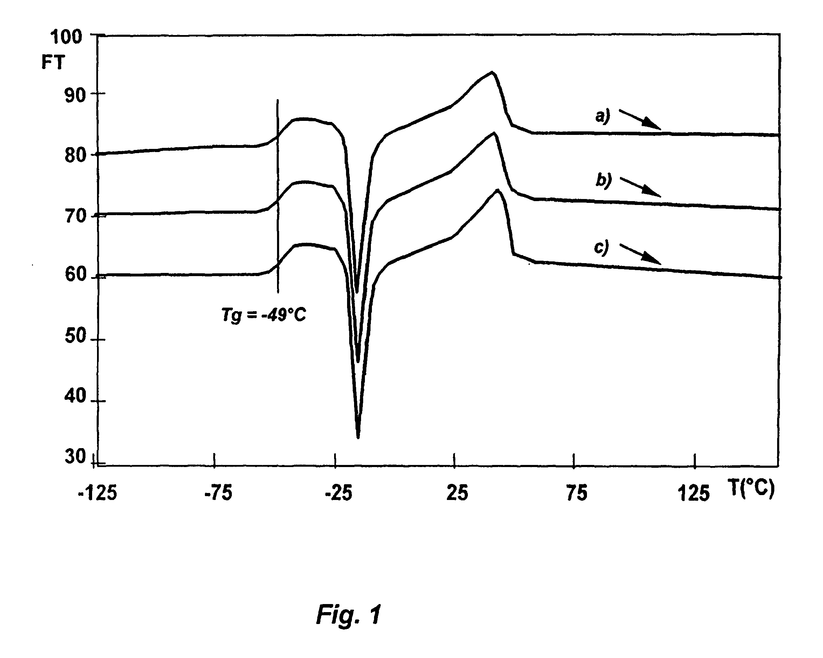

Des essais de calorimétrie différentielle à balayage (DSC) ont été effectués en vue de tester la stabilité thermique de la solution solide. Lors des essais de DSC, l'échantillon a été porté successivement à des températures de plus en plus élevées pendant quelques minutes, jusqu'à une température maximale de 160°C. La figure 1 représente les courbes de DSC, notées a), b) et c), obtenues respectivement après une exposition à 90°C, 140°C et 160°C. La température T exprimée en °C est portée en abscisse ; le flux thermique FT, exprimé en unités arbitraires, est porté en ordonnée. Les trois courbes, qui pour la commodité de la lecture, ont été décalées verticalement les unes par rapport aux autres, ont sensiblement la même allure et indiquent une température de transition vitreuse Tg d'environ -49°C.

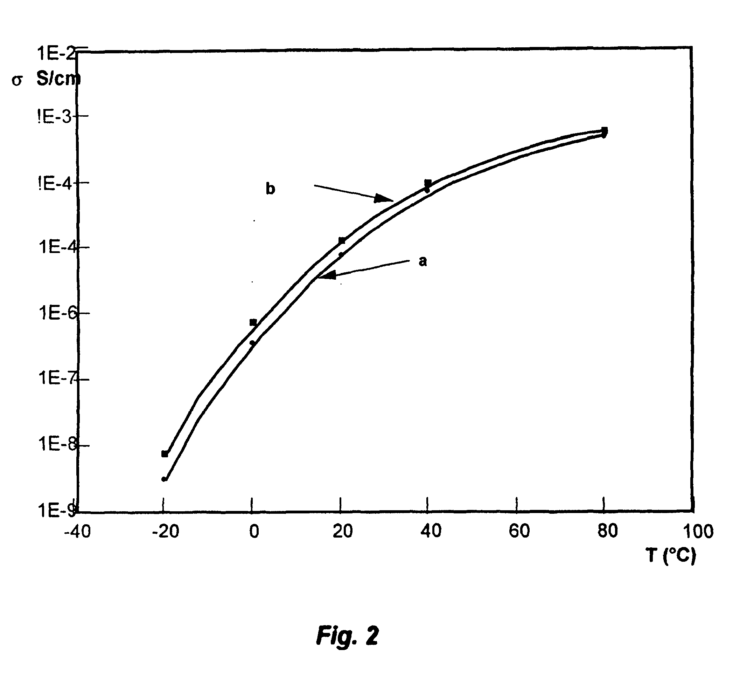

La conductivité de l'électrolyte a été déterminée par impédancemétrie et a été comparée avec celle d'un électrolyte similaire dans lequel le sel était un trifluorosulfonylimidure de lithium (LiTFSI). La figure 2 représente l'évolution de la conductivité σ, exprimée en s.cm⁻¹, en fonction de la température T, respectivement pour l'électrolyte de l'invention (courbe a) et l'électrolyte comparatif (courbe b). Il apparaît que, à la concentration en sel utilisée (O/Li = 30/1), la conductivité de LiFSI est sensiblement équivalente à celle de LiTFSI dans tout le domaine de température, qui est elle-même connue comme étant supérieure à celle du trifluorométhane sulfonate de lithium (triflate de lithium).

Ces résultats démontrent la stabilité thermique du sel de l'invention sous forme d'électrolyte polymère. Si l'on tient compte du rapport conductivité/poids moléculaire, il apparaît qu'il est très intéressant de remplacer, dans un générateur au lithium, tout ou partie des sels de lithium disponibles dans l'art antérieur, par exemple le LiTFSI, le triflate de lithium ou d'autres sels plus toxiques tels que le LiAsF₆, par le sel LiFSI de l'invention.

### EXEMPLE 3

On a dissous le sel LiFSI dans le carbonate de propylène utilisé comme solvant aprotique dans la plupart des générateurs à électrolyte liquide ainsi que comme additif dans certains électrolytes polymères pour en augmenter la conductivité ionique.

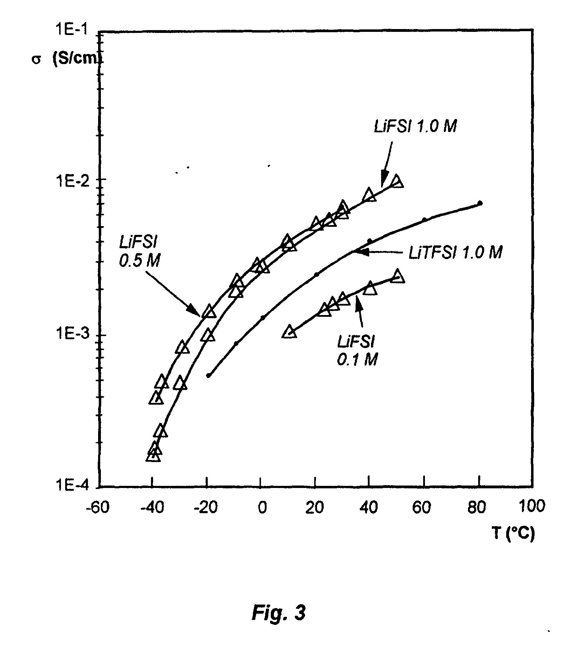

On a mesuré l'évolution de la conductivité en fonction de la température pour les trois concentrations en sel LiFSI suivantes : 1,0 molaire, 0,5 molaire et 0,1 molaire. La figure 3 représente la variation, en fonction de la température T, de la conductivité σ, exprimée en S.cm⁻¹, pour chacune des concentrations, ainsi que la variation de la conductivité pour une concentration de 1,0 en mole en sel LiTFSI à titre de comparaison.

Les valeurs de conductivité déterminées, et surtout le rapport conductivité/poids moléculaire du sel LiFSI de l'invention se comparent avantageusement à ceux du sel LiTFSI de l'art antérieur, et a fortiori, à ceux du triflate de lithium réputé moins conducteur du LiTFSI.

### EXEMPLE 4

La stabilité électrochimique du sel LiFSI a été testée dans un générateur rechargeable au lithium.

A cet effet, trois piles ont été testées ; elles sont du type :

| | | | |
|---|---|---|---|
| Li° | électrolyte polymère | cathode composite à base d'oxyde de vanadium | collecteur de nickel |

Chacune des piles est constituée par une anode de lithium ayant une épaisseur de 30 microns, un électrolyte polymère ayant une épaisseur de 30 microns et une cathode composite comportant environ 40% d'oxyde de vanadium, environ 10% de noir d'acétylène et environ 50% d'un copolymère d'oxyde d'éthylène, les proportions étant exprimées en volume. La cathode, qui a une capacité voisine de 5-6 C/cm2 est enduite sur un collecteur de nickel mince. La pile a une surface active de 3,89 cm2. Elle a été assemblée par pressage à chaud à 80°C.

Pour la pile N° 1, l'électrolyte contient le sel LiFSI, la concentration moyenne en sel de LiFSI dans la pile correspondant à un rapport O/Li de 54/1. La pile a été mise à cycler à courants constants (Id = 450 µA, puis 300 µA, et Ic = 200 µA) entre les limites de 3,3 et 1,5 volts.

Le sel de l'électrolyte de la pile N°2 est le LiTFSI avec un rapport O/Li = 30/1. Cette pile a été mise à cycler à courants constants (Id = 600 µA et Ic = 200 µA) entre les limites de 3,3 et 1,5 volts.

Le sel de l'électrolyte de la pile N°3 est le LiFSI avec un rapport O/Li de 30/1.

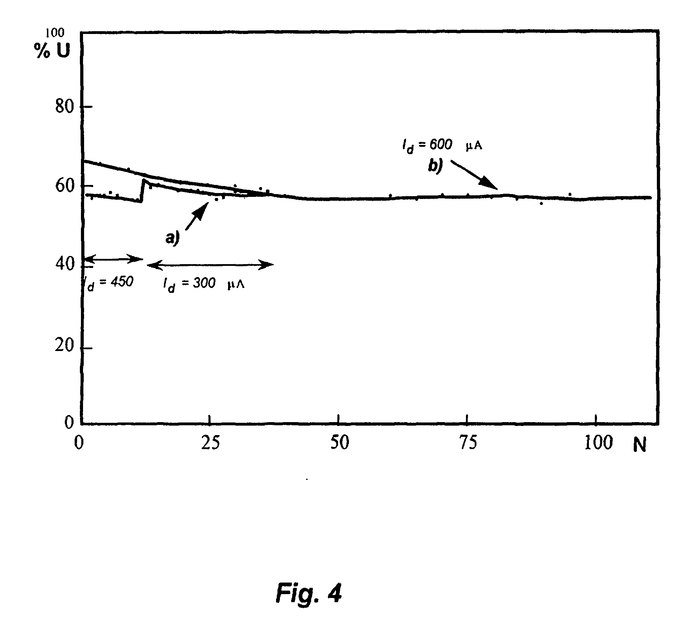

La figure 4 représente les courbes du cyclage des piles N°1 et N°2. La courbe de cyclage donne le pourcentage d'utilisation (% U) de la cathode (sur l'hypothèse de 1 atome de Li par atome de vanadium) en fonction du nombre de cycles N.

La courbe de cyclage de la pile N°1 (courbe a de la figure 4) est tout à fait comparable à celle de la pile N°2 (courbe b, figure 4) après 30 cycles charge / décharge complets, et la pente de décroissance lente de l'utilisation est sensiblement la même dans les deux cas, à la précision des mesures expérimentales près. Le taux initial d'utilisation de la cathode, légèrement plus élevé dans le cas de la courbe B), résulte d'une concentration en sel plus élevée.

La pile N°3 donne sensiblement les mêmes résultats sur 50 cycles, mais présente un taux d'utilisation identique à celui de la courbe b) obtenue par la pile N° 2 contenant LiTFSI à la même concentration.

Dans tous les cas, l'impédance initiale et l'impédance après le cyclage des piles demeurent faibles et inférieures à 15 Ω après le cyclage.

Les essais, qui ont été effectués dans des conditions exigeantes, c'est-à-dire à 60°C sur plusieurs semaines, confirment bien la stabilité thermique et électrochimique du sel de l'invention dans les conditions d'un générateur électrochimique, c'est-à-dire en l'absence d'eau et de composés chimiques susceptibles d'hydrolyser le sel. En effet, alors que la présence d'impuretés résiduelles dans le sel LiFSI utilisé aurait pu à la rigueur influencer la valeur absolue des mesures de conductivité, le comportement du sel lors du cyclage établit sans contestation la compatibilité du sel et des électrolytes réalisés selon l'invention.

### EXEMPLE 5

On a réalisé une pile rechargeable au lithium, dans laquelle l'électrolyte contient un second sel (LiTFSI) en plus un sel (LiFSI) de l'invention.

La pile est du type :

| | | | |
|---|---|---|---|
| Li° | électrolyte polymère | cathode composite à base d'oxyde de vanadium | collecteur d'aluminium |

Ses caractéristiques sont identiques à celles des piles de l'exemple 4, à l'exception du collecteur qui est l'aluminium dans le présent exemple.

La teneur en LiTFSI et en LiFSI est telle que O/Li(total) = 30/1, et que le rapport molaire FSI/TFSI soit de 8/100. Les performances en cyclage de cette pile ont été comparées avec celle d'une pile analogue ne contenant que LiTFSI. Les deux piles ont été cyclées à des courants de décharge et de charge constants (Id = 400 µA, Ic = 225 µA) entre les limites de voltage +3,3 et +1,5 Volts.

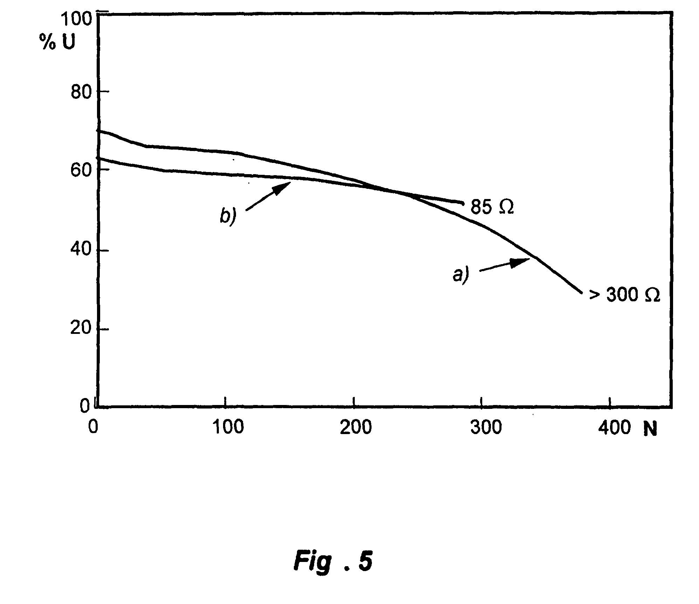

La figure 5 représente les courbes du cyclage des deux piles. La courbe de cyclage donne le pourcentage d'utilisation (%U) de la cathode (sur l'hypothèse de 1 Li par V) en fonction du nombre de cycles N. La courbe b) concerne la pile contenant le mélange de sels et la courbe a) concerne la pile ne contenant que LiTFSI. Après près de 300 cycles de déchargé profonde à 60°C, le pourcentage d'utilisation de la cathode est plus stable et surtout l'impédance après cyclage reste inférieure à 90 Ω pour la pile conforme à l'invention contenant le mélange de sels, alors que l'impédance de la pile ne contenant que LiTFSI est supérieure à 300 Ω. Il semblerait donc que l'addition de LiFSI, en principe moins stable que LiTFSI, à un électrolyte contenant LiTFSI, facilite la formation sur le collecteur en aluminium, d'un film de passivation fluoré ne gênant pas la conductivité. Le film formé semble très mince, de l'ordre de quelques dizaines ou quelques centaines de nanomètres, car l'observation au microscope à balayage (SEM) de la surface de l'aluminium après cyclage et lavage ne montre pas d'attaque significative de la surface d'aluminium, malgré la différence observée entre les valeurs d'impédance pour les deux piles comparées.

### EXEMPLE 6

On a réalisé une pile primaire au lithium fonctionnant à la température ambiante, dans laquelle l'électrolyte contient le sel (LiFSI) de l'invention, un solvant polymère similaire à celui des exemples précédents, mais plastifié par addition de carbonate de propylène (PC).

La pile est du type :

| | | | |
|---|---|---|---|
| Li° | électrolyte polymère + PC | cathode composite à base d'oxyde de manganèse | collecteur d'aluminium |

Le rapport du nombre de moles de PC au nombre de moles de sel de lithium est PC/Li = 5/1. La concentration de sel de lithium dans l'électrolyte est telle que O/Li = 30/1. La pile présente un voltage en circuit ouvert (OCV) très stable en fonction du temps sur plus de deux semaines, ce qui confirme l'absence d'auto-décharge ou de réactions secondaires.

Lorsque la pile est mise en décharge à un courant constant de 78 µA avec une limite inférieure de voltage de +2,0 volts, la courbe de décharge présente un plateau régulier typique d'une cathode de type MnO₂ dont le voltage moyen est de 2,8 volts. Le pourcentage d'utilisation (sur l'hypothèse de 1 Li par Mn) de la cathode de cette pile qui a une capacité de 5,69 C/cm² et une surface de 3,86 cm2 représente 75% de la matière active. Le résultat confirme la compatibilité électrochimique des sels de l'invention en présence d'un électrolyte liquide, utilisé dans le cas présent sous forme de plastifiant d'un électrolyte polymère.

Les facteurs de coût, de conductivité électrique et de masse molaire associés aux sels de la présente invention présentent donc des avantages importants sur les sels connus et utilisés dans l'art antérieur dans les générateurs au lithium ou au sodium. Ces avantages sont valables aussi bien pour des électrolytes liquides que pour des électrolytes polymères plastifiés ou non, ainsi que pour des générateurs mettant en oeuvre des anodes métalliques ou alliées, ou des générateurs du type rocking-chair, en particulier les générateurs du type lithium-ion, comme par exemple ceux qui utilisent LiC₆.

## Revendications

1. Procédé pour la préparation d'un composé représenté par la formule Li⁺[(ZY)₂N]⁻ dans laquelle :
- Y représente SO₂;
- chaque substituant Z représente de manière indépendante un atome de fluor ou un groupement organique perfluoré ou non qui possède éventuellement au moins une fonction polymérisable, l'un au moins des substituants Z représentant un atome de fluor ;
le procédé consistant à faire réagir l'acide correspondant H[(ZY)₂N] dans un solvant aprotique non réactif sur un sel du cation Li qui est choisi de manière à former au cours de la réaction, un acide volatil ou un acide insoluble dans le milieu réactionnel et dont la basicité est suffisamment faible pour ne pas affecter la liaison S-F, le sel du cation Li étant soit un sel qui est capable de réagir avec l'acide de départ en libérant un acide volatil et qui est choisi parmi les fluorures, les chlorures, les acétates, les trifluoroacétates, soit un sel qui est capable de réagir avec l'acide de départ en formant un acide insoluble et qui est choisi parmi les sels de diacides organiques ou de polyacides, avec une stoechiométrie telle que le produit formé soit un sel acide insoluble dans les solvants aprotiques.

2. Procédé selon la revendication 1, **caractérisé en ce que** le solvant aprotique est choisi parmi les nitriles, les nitroalcanes, les esters et les éthers.

3. Procédé selon la revendication 1, **caractérisé en ce que** le sel est choisi parmi les oxalates, les malonates, les polyacrylates, les polyméthacrylates réticulés ou non, les polyphosphates et les zéolithes.

4. Composé représenté par la formule Li⁺[(ZSO₂)₂N]⁻ dans laquelle chaque substituant Z représente de manière indépendante un atome de fluor ou un groupement organique perfluoré ou non qui possède éventuellement au moins une fonction polymérisable, l'un au moins des substituants Z représentant un atome de fluor.

5. Composé selon la revendication 4, **caractérisé en ce que** le substituant Z différent de F représente un radical perfluoroalkyle ayant de 1 à 8 atomes de carbone.

6. Composé selon la revendication 4, répondant à la formule Li⁺[(FSO₂)₂N]⁻

7. Matériau à conduction ionique qui comprend au moins un composé ionique en solution dans un solvant aprotique, **caractérisé en ce que** le composé ionique est un composé selon l'une des revendications 4 à 6.

8. Matériau selon la revendication 7, **caractérisé en ce que** le composé ionique comprend deux substituants F.

9. Matériau selon la revendication 7, **caractérisé en ce que** chaque substituant Z différent de F du composé ionique est choisi parmi les radicaux alkyles en C₁-C₃₀ ou perhaloalkyles en C₁-C₈, les radicaux aryles ou perhaloaryles en C₆-C₁₂, les radicaux arylalkyles, les radicaux oxaalkyles, azaalkyles, thiaalkyles et les hétérocycles.

10. Matériau selon la revendication 7, **caractérisé en ce que** chaque substituant Z différent de F du composé ionique est choisi parmi les radicaux qui comportent au moins une insaturation éthylénique ou un groupe condensable ou un groupe dissociable.

11. Matériau selon la revendication 7, **caractérisé en ce que** chaque substituant Z différent de F du composé ionique représente un groupe mésomorphe ou un groupe chromophore ou un polymère conducteur électronique dopé autodopé ou un alcoxysilane hydrolysable.

12. Matériau selon la revendication 7, **caractérisé en ce que** chaque substituant Z différent de F du composé ionique constitue une chaîne polymérique.

13. Matériau selon la revendication 7, **caractérisé en ce que** chaque substituant Z différent de F du composé ionique représente de manière indépendante un groupe qui comporte un piège à radicaux libres tel qu'un phénol encombré, ou une quinone ; ou un dipôle dissociant tel qu'une amide ou un nitrile ; ou un couple rédox tel qu'un disulfure, un thioamide, un ferrocène, une phénothiazine, un groupe bis(dialkylaminoaryle), un nitroxyde, un imide aromatique ; ou un ligand complexant ; ou un zwitterion.

14. Matériau selon la revendication 7 **caractérisé en ce que** chaque substituant Z différent de F du composé ionique est choisi parmi les groupes R³-CFX-, R³-O-CF₂-CFX-, R¹R²N-CO-CFX- ou R¹R²N-SO₂-(CF₂)ₙ-CFX- avec n = 1, 2 ou 3, dans lesquels :
- X représente F, Cl, H ou RF ;
- les radicaux R¹, R² et R³, identiques ou différents, sont choisis parmi les radicaux organiques non perfluorés polymérisables ;
- R_{F} est choisi parmi les radicaux perfluoroalkyles et les radicaux perfluoroaryles.

15. Matériau selon la revendication 14, **caractérisé en ce que** les radicaux R_{F} sont choisis parmi les radicaux perfluoroalkyle ayant de 1 à 8 atomes de carbone ou parmi les radicaux perfluoroaryle ayant de 6 à 8 atomes de carbone.

16. Matériau selon la revendication 14, **caractérisé en ce que** les groupements organiques non perfluorés polymérisables R¹, R² et R³ sont choisis parmi les radicaux organiques qui comportent des doubles liaisons, ou parmi les radicaux qui comportent des fonctions oxirane ou oxétane, ou parmi les radicaux qui comportent des fonctions alcool, thiol, arylamine, isocyanate ou trialcoxysilane, ou parmi les radicaux qui comportent des fonctions permettant une électropolymérisation.

17. Matériau selon la revendication 7, **caractérisé en ce que** la fonction polymérisable du substituant Z différent de F est une fonction polymérisable par voie radicalaire, par voie anionique ou par une réaction de type Vandenberg.

18. Matériau selon la revendication 7, **caractérisé en ce que** le solvant est un solvant liquide aprotique, choisi parmi les éthers linéaires et les éthers cycliques, les esters, les nitriles, les dérivés nitrés, les amides, les sulfones, les sulfolanes et les sulfamides.

19. Matériau selon la revendication 7, **caractérisé en ce que** le solvant est un polymère solvatant, réticulé ou non, portant ou non des groupements ioniques greffés.

20. Matériau selon la revendication 19, **caractérisé en ce que** le polymère solvatant est choisi parmi les polyéthers de structure linéaire, peigne ou à blocs, formant ou non un réseau, à base de poly(oxyde d'éthylène), les copolymères contenant le motif oxyde d'éthylène ou oxyde de propylène ou allylglycidyléther, les polyphosphazènes, les réseaux réticulés à base de polyéthylène glycol réticulé par des isocyanates, les réseaux obtenus par polycondensation et portant des groupements qui permettent l'incorporation de groupements réticulables et les copolymères à blocs dans lesquels certains blocs portent des fonctions qui ont des propriétés rédox.

21. Matériau selon la revendication 7, **caractérisé en ce que** le solvant est un mélange d'un solvant liquide aprotique et d'un solvant polymère solvatant.

22. Matériau selon la revendication 7, **caractérisé en ce que** le solvant est constitué essentiellement par un solvant liquide aprotique et un solvant polymère polaire non solvatant comprenant des unités contenant au moins un hétéroatome choisi parmi le soufre, l'oxygène, l'azote et le fluor.

23. Matériau selon la revendication 21, **caractérisé en ce que** le polymère polaire non solvatant est choisi parmi un poly(acrylonitrile), un poly (fluorovinylidène) ou une poly (N-vinylpyrrolidone).

24. Matériau selon la revendication 7 , **caractérisé en ce qu'**il contient en outre au moins un second sel.

25. Matériau selon la revendication 24, **caractérisé en ce que** le second sel est choisi parmi les perfluoroalcanesulfonates, les bis(perfluoroalkylsulfonyl)imidures, les bis (perfluoroalkylsulfonyl)méthanes, les tris (perfluoroalkylsulfonyl)méthanes, ou leurs homologues dans lesquels au moins un groupe sulfonyle est remplacé par un groupe carbonyle.

26. Matériau selon la revendication 7, **caractérisé en ce qu'**il contient en outre un plastifiant et/ou une charge.

27. Générateur électrochimique au lithium, **caractérisé en ce que** l'électrolyte est un matériau selon la revendication 7.

28. Générateur selon la revendication 27, **caractérisé en ce que** l'électrode négative est constituée par du lithium métallique, un alliage de lithium ou du lithium ionique inséré.

29. Générateur selon la revendication 27, **caractérisé en ce que** le collecteur de la cathode est en aluminium.

30. Supercapacité du type carbone ou du type polymère rédox, comprenant un matériau selon l'une des revendications 7 à 26 comme électrolyte.

31. Procédé pour le dopage p ou n d'un polymère à conduction électronique, **caractérisé en qu'**il met en oeuvre un matériau selon l'une des revendications 7 à 26.

32. Dispositif électrochrome, dans lequel l'électrolyte est un matériau selon l'une des revendications 7 à 26.

## Claims

1. A process for the preparation of a compound represented by the formula Li⁺[(ZY)₂N]⁻, in which :
- Y denotes SO₂ ;
- each substituent Z independently denotes a fluorine atom or an optionally perfluorinated organic group which optionally contains at least one polymerizable functional group, at least one of the substituents Z denoting a fluorine atom ;
said process consisting in reacting the corresponding acid, H[(ZY)₂N], in an unreactive aprotic solvent, with a salt of the cation Li which is chosen so as to form, during the reaction, a volatile acid or an acid which is insoluble in the reaction mixture and which has a basicity sufficiently weak not to affect the S-F bond, the salt of the Li cation being a salt which is capable of releasing a volatile acid which is chosen from fluorides, chlorides, acetates and trifluoroacetates, or a salt which is capable of reacting with the staring acid to form an insoluble acid and which is chosen from the salts of organic diacids or polyacids, by choosing a stoichiometry such that the product formed is an acid salt which is insoluble in aprotic solvents.

2. A process according to Claim 1, **characterized in that** the aprotic solvent is chosen from nitriles, nitroalkanes, esters and ethers.

3. A process according to Claim 1, **characterized in that** the salt is chosen from oxalates, malonates, polyacrylates, polymethacrylates, crosslinked or otherwise, polyphosphates and zeolites.

4. A compound represented by the formula Li⁺[(ZSO₂)₂N]⁻, in which each substituent Z independently denotes a fluorine atom or an optionally perfluorinated organic group which optionally contains at least one polymerizable functional group, at least one of the substituents Z denoting a fluorine atom.

5. A compound according to Claim 4, **characterized in that** substituent Z which is different from F represents a perfluoroalkyl radical containing from 1 to 8 carbon atoms.

6. A compound according to claim 4, represented by the formula Li⁺[(FSO₂)₂N]⁻.

7. An ionically conductive material which includes at least one ionic compound in solution in an aprotic solvent, **characterized in that** the ionic compound is a compound according to any of claims 4-6.

8. A material according to claim 7, **characterized in that** the ionic compound has two F substituents.

9. A material according to claim 7, **characterized in that** each substituent Z other than F of the ionic compound is chosen from C₁-C₃₀ alkyl or C₁-C₈ perhaloalkyl radicals, C₆-C₁₂ aryl or perhaloaryl radicals, arylalkyl radicals, oxaalkyl, azaalkyl, thiaalkyl radicals and heterocyclic rings.

10. A material according to Claim 7, **characterized in that** each substituent Z other than F of the ionic compound is chosen from radicals which contain at least one ethylenic unsaturation or a condensable group or a dissociable group.

11. A material according to Claim 7, **characterized in that** each substituent Z other than F of the ionic compound denotes a mesomorphic group or a chromophore group or a doped or autodoped electronic conductor polymer or a hydrolysable alkoxysilane.

12. A material according to Claim 7, **characterized in that** each substituent Z other than F of the ionic compound forms a polymeric chain.

13. A material according to Claim 7, **characterized in that** each substituent Z other than F of the ionic compound independently denotes a group which contains a scavenger for free radicals such as a hindered phenol or a quinone; or a dissociating dipole such as an amide or a nitrile; or a redox couple such as a disulphide, a thioamide, a ferrocene, a phenothiazine, a bis(dialkylaminoaryl) group, a nitroxide, an aromatic imide; or a complexing ligand; or a zwitterion.

14. A material according to Claim 1, **characterized in that** each substituent Z other than F of the ionic compound is chosen from the groups R³-CFX-, R³-O-CF₂-CFX-, R¹R²N-CO-CFX- and R¹R²N-SO₂-(CF₂)ₙ-CFX- with n = 1, 2 or 3, in which:
- X denotes F, Cl, H or R_{F};
- the radicals R¹, R² and R³, which are identical or different, are chosen from polymerizable nonperfluorinated organic radicals;
- R_{F} is chosen from perfluoroalkyl radicals and perfluoroaryl radicals.

15. A material according to Claim 14, **characterized in that** the radicals R_{F} are chosen from perfluoroalkyl radicals containing from 1 to 8 carbon atoms or from perfluoroaryl radicals containing from 6 to 8 carbon atoms.

16. A material according to Claim 14, **characterized in that** the polymerizable nonperfluorinated organic groups R¹, R² and R³ are chosen from organic radicals containing double bonds or from radicals containing oxirane or oxetane functional groups or from radicals containing alcohol, thiol, arylamine, isocyanate or trialkoxysilane functional groups or from radicals containing functional groups permitting an electropolymerization.

17. A material according to Claim 7, **characterized in that** the polymerizable functional group of the substituent Z other than F is a functional group polymerizable by a radical route, by an anionic route or by a reaction of Vandenberg type.

18. A material according to Claim 7, **characterized in that** the solvent is an aprotic liquid solvent chosen from linear ethers and cyclic ethers, esters, nitriles, nitro derivatives, amides, sulphones, sulpholanes and sulphamides.

19. A material according to Claim 7, **characterized in that** the solvent is an optionally crosslinked solvating polymer optionally carrying grafted ionic groups.

20. A material according to Claim 19, **characterized in that** the solvating polymer is chosen from polyethers of linear, comb or block structure, optionally forming a network, based on poly(ethylene oxide), copolymers containing the ethylene oxide or propylene oxide or allyl glycidyl ether unit, polyphosphazenes, crosslinked networks based on polyethylene glycol crosslinked with isocyanates, networks obtained by polycondensation and bearing groups which allow the incorporation of crosslinkable groups and block copolymers in which some blocks carry functional groups which have redox properties.

21. A material according to Claim 7, **characterized in that** the solvent is a mixture of an aprotic liquid solvent and of a solvating polymer solvent.

22. A material according to Claim 7, **characterized in that** the solvent consists essentially of an aprotic liquid solvent and a nonsolvating polar polymer solvent including units containing at least one heteroatom chosen from sulphur, oxygen, nitrogen and fluorine.

23. A material according to Claim 21, **characterized in that** the nonsolvating polar polymer is chosen from a poly(acrylonitrile), a poly(fluorovinylidene) and a poly(N-vinylpyrrolidone).

24. A material according to Claim 7, **characterized in that** it additionally contains at least one second salt.

25. A material according to Claim 24, **characterized in that** the second salt is chosen from perfluoroalkanesulphonates, bis(perfluoroalkylsulphonyl)imides, bis(perfluoroalkylsulphonyl)methanes, tris(perfluoroalkylsulphonyl)methanes or their homologues in which at least one sulphonyl group is replaced by a carbonyl group.

26. A material according to Claim 7, **characterized in that** it additionally contains a plasticizer and/or a filler.

27. A lithium electrochemical generator **characterized in that** the electrolyte is a material according to Claim 7.

28. A generator according to Claim 27, **characterized in that** the negative electrode consists of metallic lithium, a lithium alloy or intercalated ionic lithium.

29. A generator according to Claim 27, **characterized in that** the collector of the cathode is made of aluminum.

30. A supercapacity of the carbon type or of the redox polymer type, including a material according to one of Claims 7 to 26 as electrolyte.

31. A process for the p or n doping of an electronically conductive polymer, **characterized in that** it uses a material according to any of claims 7 to 26.

32. An electrochromic device in which the electrolyte is a material according to one of Claims 7 to 26.

## Patentansprüche

1. Verfahren zur Herstellung einer Verbindung der Formel Li⁺[(ZY)₂N]⁻, worin:
- Y für SO₂ steht;
- die Substituenten Z jeweils unabhängig voneinander für ein Fluoratom oder eine gegebenenfalls perfluorierte organische Gruppierung steht, die gegebenenfalls zumindest eine polymerisierbare Funktionalität aufweist, wobei zumindest einer der Substituenten Z für ein Fluoratom steht;
wobei das Verfahren darin besteht, die entsprechende Säure H[(ZY)₂N] in einem nicht reaktiven, aprotischen Lösungsmittel mit einem Salz des Li-Kations umzusetzen, das so gewählt ist, dass im Verlauf der Reaktion eine flüchtige Säure oder eine im Reaktionsmedium unlösliche Säure gebildet wird, und dessen Basizität ausreichend gering ist, um die Bindung S-F nicht zu beeinflussen, worin das Salz des Li-Kations entweder ein Salz ist, das mit der Ausgangssäure unter Freisetzung einer flüchtigen Säure reagieren kann und aus Fluoriden, Chloriden, Acetaten und Trifluoracetaten ausgewählt ist, oder ein Salz ist, das mit der Ausgangssäure unter Bildung einer unlöslichen Säure reagieren kann und aus Salzen organischer Disäuren oder Polysäuren ausgewählt ist, und zwar mit einer solchen Stöchiometrie, dass das gebildete Produkt ein in aprotischen Lösungsmitteln unlösliches saures Salz ist.

2. Verfahren nach Anspruch 1, **dadurch gekennzeichnet, dass** das aprotische Lösungsmittel aus Nitrilen, Nitroalkanen, Estern und Ethern ausgewählt ist.

3. Verfahren nach Anspruch 1, **dadurch gekennzeichnet, dass** das Salz aus Oxalaten, Malonaten, Polyacrylaten, gegebenenfalls vernetzten Polymethacrylaten, Polyphosphaten und Zeolithen ausgewählt ist.

4. Verbindung der Formel Li⁺[(ZSO₂)₂N]⁻, worin die Substituenten Z jeweils unabhängig voneinander für ein Fluoratom oder eine gegebenenfalls perfluorierte organische Gruppierung steht, die gegebenenfalls zumindest eine polymerisierbare Funktionalität aufweist, wobei zumindest einer der Substituenten Z für ein Fluoratom steht.

5. Verbindung nach Anspruch 4, **dadurch gekennzeichnet, dass** der Substituent Z, der nicht F ist, für einen Perfluoralkylrest mit 1 bis 8 Kohlenstoffatomen steht.

6. Verbindung nach Anspruch 4, die der Formel Li⁺[(FSO₂)₂N]⁻ entspricht.

7. lonenleitendes Material, das zumindest eine ionische Verbindung in Lösung in einem aprotischen Lösungsmittel umfasst, **dadurch gekennzeichnet, dass** die ionische Verbindung eine Verbindung nach einem der Ansprüche 4 bis 6 ist.

8. Material nach Anspruch 7, **dadurch gekennzeichnet, dass** die ionische Verbindung zwei Substituenten umfasst.

9. Material nach Anspruch 7, **dadurch gekennzeichnet, dass** jeder Substituent Z der ionischen Verbindung, der nicht F ist, aus C₁-C₃₀-Alkylresten oder C₁-C₈-Perhalogenalkylresten, C₆-C₁₂-Aryl- oder -Perhalogenarylresten, Arylalkylresten, Oxaalkyl-, Azaalkyl-, Thiaalkylresten und heterozyklischen Verbindungen ausgewählt ist.

10. Material nach Anspruch 7, **dadurch gekennzeichnet, dass** jeder Substituent Z der ionischen Verbindung, der nicht F ist, aus Resten ausgewählt ist, die zumindest eine ethylenische Unsättigung oder eine kondensierbare Gruppe oder eine dissoziierbare Gruppe umfassen.

11. Material nach Anspruch 7, **dadurch gekennzeichnet, dass** jeder Substituent Z der ionischen Verbindung, der nicht F ist, für eine mesomorphe Gruppe oder eine chromophore Gruppe oder ein dotiertes oder autodotiertes elektronenleitendes Polymer oder ein hydrolysierbares Alkoxysilan steht.

12. Material nach Anspruch 7, **dadurch gekennzeichnet, dass** jeder Substituent Z der ionischen Verbindung, der nicht F ist, eine Polymerkette darstellt.

13. Material nach Anspruch 7, **dadurch gekennzeichnet, dass** die Substituenten Z der ionischen Verbindung, die nicht F sind, jeweils unabhängig voneinander für eine Gruppe stehen, die einen Radikalfänger, wie z.B. ein sterisch gehindertes Phenol oder ein Chinon; einen dissoziierenden Dipol, wie z.B. ein Amid oder ein Nitril; ein Redox-Paar, wie z.B. ein Disulfid, ein Thioamid, ein Ferrocen, ein Phenothiazin, eine Bis(dialkylaminoaryl)gruppe, ein Nitroxid oder ein aromatisches Imid; einen komplexierenden Liganden; oder ein Zwitterion umfasst.

14. Material nach Anspruch 7, **dadurch gekennzeichnet, dass** die Substituenten der ionischen Verbindung, die nicht F sind, jeweils aus den Gruppen R³-CFX-, R³-O-CF₂-CFX-, R¹R²N-CO-CFX- oder R¹R²N-SO₂-(CF₂)ₙ-CFX-, wobei n = 1, 2 oder 3 ist, ausgewählt sind, worin:
- X für F, Cl, H oder R_{F} steht;
- die Reste R¹, R² und R³, die gleich oder unterschiedlich sein können, aus nicht perfluorierten, polymerisierbaren organischen Resten ausgewählt sind;
- R_{F} aus Perfluoralkylresten und Perfluorarylresten ausgewählt ist.

15. Material nach Anspruch 14, **dadurch gekennzeichnet, dass** die Reste R_{F} aus Perfluoralkylresten mit 1 bis 8 Kohlenstoffatomen oder aus Perfluorarylresten mit 6 bis 8 Kohlenstoffatomen ausgewählt sind.

16. Material nach Anspruch 14, **dadurch gekennzeichnet, dass** die nicht perfluorierten, polymerisierbaren organischen Gruppierungen R¹, R² und R³ aus organischen Resten, die Doppelbindungen umfassen, oder aus Resten, die Oxiran- oder Oxetan-Funktionalitäten umfassen, oder aus Resten, die Alkohol-, Thiol-, Arylamin-, Isocyanat- oder Trialkoxysilan-Funktionalitäten umfassen, oder aus Resten, die Elektropolymerisation ermöglichende Funktionalitäten umfassen, ausgewählt sind.

17. Material nach Anspruch 7, **dadurch gekennzeichnet, dass** die polymerisierbare Funktionalität des Substituenten Z, der nicht F ist, eine radikalisch, anionisch oder durch Vandenberg-Reaktion polymerisierbare Funktion ist.

18. Material nach Anspruch 7, **dadurch gekennzeichnet, dass** das Lösungsmittel ein flüssiges, aprotisches Lösungsmittel, ausgewählt aus linearen Ethern und zyklischen Ethern, Estern, Nitrilen, nitrierten Derivaten, Amiden, Sulfonen, Sulfolanen und Sulfamiden, ist.

19. Material nach Anspruch 7, **dadurch gekennzeichnet, dass** das Lösungsmittel ein gegebenenfalls vernetztes solvatisierendes Polymer ist, das gegebenenfalls aufgepfrofte ionische Gruppierungen trägt.

20. Material nach Anspruch 19, **dadurch gekennzeichnet, dass** das solvatisierende Polymer aus Polyethern mit linearer, Kamm- oder Block-Struktur, die gegebenenfalls ein Netzwerk bilden, auf Basis von Poly(ethylenoxid), Copolymeren, die Ethylenoxid- oder Propylenoxid- oder Allylglycidylether-Motive aufweisen, Polyphosphazenen, vernetzten Netzwerken auf Basis von mit Isocyanaten vernetztem Polyethylenglykol, Netzwerken, die durch Polykondensation erhalten werden und Gruppierungen tragen, die den Einbau von vernetzbaren Gruppierungen ermöglichen, sowie BlockCopolymeren, in denen bestimmte Blöcke Funktionalitäten tragen, die Redox-Eigenschaften aufweisen, ausgewählt ist.

21. Material nach Anspruch 7, **dadurch gekennzeichnet, dass** das Lösungsmittel ein Gemisch aus einem flüssigen aprotischen Lösungsmittel und einem solvatisierenden Polymer-Lösungsmittel ist.

22. Material nach Anspruch 7, **dadurch gekennzeichnet, dass** das Lösungsmittel im Wesentlichen aus einem flüssigen aprotischen Lösungsmittel und einem nicht solvatisierenden, polaren Polymer-Lösungsmittel besteht, das Einheiten umfasst, die zumindest ein Heteroatom, ausgewählt aus Schwefel, Sauerstoff, Stickstoff und Fluor, enthalten.

23. Material nach Anspruch 21, **dadurch gekennzeichnet, dass** das nicht solvatisierende polare Polymer aus Poly(acrylnitril), Poly(fluorvinyliden) und einem Poly-(N-vinylpyrrolidon) ausgewählt ist.

24. Material nach Anspruch 7, **dadurch gekennzeichnet, dass** es weiters zumindest ein zweites Salz enthält.

25. Material nach Anspruch 24, **dadurch gekennzeichnet, dass** das zweite Salz aus Perfluoralkansulfonaten, Bis(perfluoralkylsulfonyl)imiden, Bis(perfluoralkylsulfonyl)methanen, Tris(perfluoralkylsulfonyl)methanen oder Homologen davon, in denen zumindest eine Sulfonylgruppe durch eine Carbonylgruppe ersetzt ist, ausgewählt ist.

26. Material nach Anspruch 7, **dadurch gekennzeichnet, dass** es darüber hinaus einen Weichmacher und/oder eine Ladung enthält.

27. Elektrochemischer Lithiumgenerator, **dadurch gekennzeichnet, dass** der Elektrolyt ein Material nach Anspruch 7 ist.

28. Generator nach Anspruch 27, **dadurch gekennzeichnet, dass** die negative Elektrode aus metallischem Lithium, einer Lithiumlegierung oder aus eingelagerten Lithiumionen besteht.

29. Generator nach Anspruch 27, **dadurch gekennzeichnet, dass** der Kollektor der Kathode aus Aluminium ist.

30. Superkapazität vom Kohlenstoff-Typ oder vom Redox-Polymer-Typ, umfassend ein Material nach einem der Ansprüche 7 bis 26 als Elektrolyt.

31. Verfahren zur p- oder n-Dotierung eines ionenleitenden Polymers, **dadurch gekennzeichnet, dass** ein Material nach einem der Ansprüche 7 bis 26 verwendet wird.

32. Elektrochrome Vorrichtung, worin der Elektrolyt ein Material nach einem der Ansprüche 7 bis 26 ist.
